# EUROPÄISCHE PATENTSCHRIFT

(11) **EP 2 537 041 B1**
(45) Veröffentlichungstag und Bekanntmachung des Hinweises auf die Patenterteilung: **09.04.2014**
(21) Anmeldenummer: 10706519.5
(22) Anmeldetag: 19.02.2010
(51) Int. Cl.: G01R 33/28, G02B 27/40, G01R 33/565, G01R 33/567, A61B 17/00

(54) **MR-APPARATUR MIT AUTOFOKUSSIERENDEM BEWEGUNGSMONITORSYSTEM**
MR-APPARATUS WITH AUTOFOCUSING MOTION MONITOR SYSTEM
APPAREILLAGE À RÉSONANCE MAGNÉTIQUE COMPRENANT UN SYSTÈME DE CONTRÔLE DE MOUVEMENT À MISE AU POINT AUTOMATIQUE

(43) Veröffentlichungstag der Anmeldung: 26.12.2012
(73) Patentinhaber: Albert-Ludwigs-Universität Freiburg, 79085 Freiburg (DE)
(72) Erfinder: WALLRABE, Ulrike, 76228 Karlsruhe (DE); SCHNEIDER, Florian, 79276 Reute (DE); ZAITSEV, Maxim, 79117 Freiburg (DE); MACLAREN, Julian, 79100 Freiburg (DE); HERBST, Michael, 79104 Freiburg (DE)
(74) Vertreter: Kohler Schmid Möbus
(86) Internationale Anmeldenummer: PCT/EP2010/001050
(87) Internationale Veröffentlichungsnummer: WO 2011/100993

(56) Entgegenhaltungen:
- US-A1- 2009 209 846
- SCHNEIDER F. ET AL: "A low cost adaptive silicone membrane lens", JOURNAL OF OPTICS A: PURE AND APPLIED OPTICS, Bd. 10, 25. März 2008 (2008-03-25), Seiten 1-5, XP002608330, in der Anmeldung erwähnt
- SCHNEIDER F. ET AL: "Optical quality and system behaviour of adaptive siliconemembranelenses", PROCEEDINGS MIKROSYSTEMTECHNIK KONGRESS 2009, 12. Oktober 2009 (2009-10-12), Seiten 1-4, XP002608331, Berlin
- Anonymous: "Highlights am Institut für Mikrosystemtechnik", Uni Freiburg Newsletter, Nr. 1 März 2010 (2010-03), Seiten 1-20, XP002608332, Gefunden im Internet: URL:http://www.imtek.de/www.imtek.de/conte nt/upload/news/2010/imtek-newsletter_nr._1 _2010.pdf [gefunden am 2010-11-04]
- QIN LEI ET AL: "Prospective head-movement correction for high-resolution MRI using an in-bore optical tracking system.", MAGNETIC RESONANCE IN MEDICINE : OFFICIAL JOURNAL OF THE SOCIETY OF MAGNETIC RESONANCE IN MEDICINE / SOCIETY OF MAGNETIC RESONANCE IN MEDICINE OCT 2009 LNKD- PUBMED:19526503, Bd. 62, Nr. 4, Oktober 2009 (2009-10), Seiten 924-934, XP002608333, ISSN: 1522-2594 in der Anmeldung erwähnt
- Anonymous: "MRC Medical Products", MRC Systems GmbH Internet Article, 2004, XP002608334, Gefunden im Internet: URL:http://web.archive.org/web/20040701182 734/www.mrc-systems.de/pdfs/competenceinmr i_a4_leaflet.pdf [gefunden am 2010-11-05]
- Anonymous: "MR-kompatible Kamera, Benutzerhandbuch", MRC Systems GmbH Internet Article, 2009, Seiten 1-5, XP002608335, Gefunden im Internet: URL:http://www.mrc-systems.de/pdfs/manual_ mrcam_12M.pdf [gefunden am 2010-11-05]

## Beschreibung

### Hintergrund der Erfindung

Die Erfindung betrifft eine Magnetresonanz (=MR) Apparatur mit einem MR-Magnetsystem zur Erzeugung eines homogenen Magnetfelds, das eine Bohrung zur Aufnahme eines Messobjektes aufweist, sowie mit einem optischen Bewegungsmonitorsystem zur Erfassung von Bewegungen des Messobjektes, umfassend eine innerhalb der Bohrung angeordnete, optische Kamera mit einem Kameraobjektiv, sowie eine Vorrichtung zur Übertragung von Monitoringdaten an einen außerhalb des MR- Magnetsystems vorgesehenen Bildwandler.

Eine solche Apparatur ist aus US 2009/0209846 A1, sowie aus Qin et al. [Magn. Reson. Med., 2009; 62: 924-934] bekannt.

Bewegungen von Messobjekten, vor allem von Lebewesen wie etwa Patienten während eines Magnetresonanz (=MR) -scans führen zu Artefakten, welche die Daten etwa für die medizinische Verwendung oft nutzlos machen. Insbesondere im medizinischen Bereich führt dies zu einer Erschwerung der Diagnostik und Reduktion des Patientendurchsatzes.

Es wurden bereits unterschiedliche Systeme für optisches Bewegungsmonitoring entwickelt, welche jedoch aufgrund der erschwerten Handhabung nicht kommerzialisiert wurden.

Die erste erfolgreiche 3 D Echtzeit Bewegungskorrektur wurde von Hennig, Zaitsev und Speck et. al. [Zaitsev M, Dold C, Sakas G, Hennig J, und Speck O. Neuroimage, 2006; 31(3): 1038-1050 und Speck O, Hennig J, und Zaitsev M. Magn. Reson. Mater. Phy., 2006; 19(2): 55-61] entwickelt.

Das System zur Erfassung der Bewegung befand sich dabei außerhalb der Bohrung innerhalb des MRT-Raums (Abstand ca. 4 m). Der Marker zur Bewegungserfassung befand sich dabei am Kopf des Patienten und wurde direkt angepeilt und erfasst. Zur Korrektur der Bewegung wurden die Monitoringdaten einer Stereokamera direkt von einer Hardwarekontrolleinheit an den MRT-Bildwandler übergeben und korrigiert.

Ähnliche Lösungsansätze sind aus US 7,498,811 B2, US 2005/0054910 A1 und US 2007/0280508 A1 bekannt.

Aufgrund des langen optischen Pfades zwischen Stereokamera und Marker ist die Justage und Kalibrierung derartiger optischer Systeme sehr aufwendig.

Aus diesem Grund wurde vom Qin et al. [Magn. Reson. Med., 2009; 62: 924-934] und Aksoy et al. [Aksoy M, Newbould R, Straka M, Holdsworth S, Skare S, Santos J, Bammer R. Proceedings of the 16th Scientific Meeting, Int. Soc. Magn. Reson. Med., Toronto, Canada, 2008, p 3120] eine MR-kompatible Kamera entwickelt, welche sich im Magnet-Kern direkt oberhalb des Markers befindet. Die permanente Fokussierung des Markers wurde mit einer kleinen Lochblende, welche eine sehr große Schärfentiefe aufweist, realisiert. Der große Schärfenbereich, führt jedoch zur Reduktion der optischen Auflösung und somit zu einer deutlichen Verringerung der Messgenauigkeit.

US 2009/0209846 A1 stellt nun eine Weiterentwicklung dieser Idee dar. Dabei wurden zwei CCD Kameras mittels Kupferfolie vom Hochfrequenzfeld der MR-Apparatur abgeschirmt und auf einer der Magnetspulen einer 1,5T MR-Apparatur angebracht. Aus der CCD Kamera wurden magnetische Komponenten weitgehend, jedoch nicht vollständig, entfernt. Auch hier wurde eine Lochblende für einen großen Schärfenbereich verwendet.

Handelsübliche Autofokussysteme, wie sie etwa in Barcode-Lesegeräten eingebaut werden, verwenden Tauchspulenantriebe. Diese aufgrund der magnetischen Materialien nicht MR-kompatiblen Aktoren sind für den Einsatz innerhalb im Magnetkern eines Magneten vollkommen ungeeignet.

Schneider, Mueller und Wallrabe entwickelten 2008 eine Silikon-Membran-Linse [Journal of Optics A - Pure and Applied Optics, 2008] mit dem Ziel, eine preisgünstige und kompakte adaptive Linse für Mobil-Elektronik-Geräte, wie etwa Mobiltelefone, zu erhalten.

### Kurze Beschreibung der Erfindung

Aufgabe der vorliegenden Erfindung ist es demgegenüber, eine MR-Apparatur der eingangs beschriebenen Art mit einem optischen Bewegungsmonitoringsystem zur Erfassung von unkontrollierten Bewegungen des Messobjekts, insbesondere von zu untersuchenden Patienten, während eines MR-Scans zu entwickeln, welches innerhalb der Bohrung des MR-Magnetsystems angeordnet ist, die MR-Messung nicht negativ beeinflusst und trotz eines großen Schärfenbereichs eine hohe optische Auflösung aufweist.

Diese Aufgabe wird erfindungsgemäß auf ebenso überraschend einfache wie wirkungsvolle Weise dadurch gelöst, dass das Kameraobjektiv ein Autofokussystem aus nicht-magnetischem Material umfasst, dass das Autofokussystem Hochfrequenz (=HF) Felder weder absorbiert noch reflektiert noch emittiert, dass im Autofokussystem mindestens eine adaptive Linse angeordnet ist, dass die adaptive Linse eine Silikon-Membran-Linse ist oder aus einem Voll-Polymer hergestellt ist, und dass die adaptive Linse eine Linsenkammer, eine Pumpkammer und einen piezoelektronischen Biegewandler umfasst, die jeweils aus nicht-magnetischem Material hergestellt sind, wobei zur Brennweiteneinstellung mittels des Biegewandlers Flüssigkeit von der einen kammer in die andere kammer verdrängt werden kann.

Im Gegensatz zum Stand der Technik wird beim erfindungsgemäßen System der Marker permanent mit einem Autofokus scharf gestellt. Durch die Autofokussierung des Markers kann die Schärfentiefe deutlich reduziert und somit die optische Auflösung bzw. die Messgenauigkeit wesentlich erhöht werden. Die gemessenen Bewegungsparameter werden für eine Echtzeitkorrektur der MRT-Daten verwendet. Zur Visualisierung des bewegten Körpers befindet sich das System innerhalb des Magnetkerns. Dabei muss die Kompatibilität der Einzelkomponenten wie KameraChip und Autofokusoptik im Hinblick auf das starke Magnetfeld (bis zu 11,7 T), den geschalteten Gradientenfeldern sowie des starken Radiofrequenzfelds üblicherweise 64 bis 500 MHz) gewährleistet sein.

Im Autofokussystem ist mindestens eine adaptive Linse angeordnet. Eine solche adaptive Linse erlaubt eine besonders kompakte Bauweise des Autofokussystems. Insbesondere in MR-Magnetsystemen mit sehr hohen Magnetfeldern ist die Bohrung sehr klein ausgeführt, so dass zum Unterbringen eines Kameraobjektivs nur sehr wenig Raum zur Verfügung steht.

Bei einer Ausführungsform ist die adaptive Linse eine Silikon-Membran-Linse. Alle Komponenten der Linse bestehen aus nicht-magnetischem Material. Daher eignet sie sich besonders zum Einsatz in einer MR-Apparatur.
Eine alternative Möglichkeit wäre, die adaptive Linse aus einem Voll-Polymer statt aus Silikon herzustellen.

Die adaptive Linse umfasst eine Linsenkammer, eine Pumpkammer und einen piezoelektronischen Biegewandler, die jeweils aus nicht-magnetischem Material hergestellt sind. Dies erlaubt die Veränderung der Brennweite der Linse ohne Elektromotoren.

### Beschreibung der bevorzugten Ausführungsformen

Eine alternative Möglichkeit wären piezoelektronische Wanderwellenmotoren, wie sie in Spiegelreflex-Kameras verbaut werden. Auch diese sind MR-kompatibel.

Zu bevorzugen ist es, wenn die adaptive Linse mit einer optisch transparenten Flüssigkeit, insbesondere einem Immersionsöl, gefüllt ist. Die Flüssigkeit sorgt für den notwendigen Brechungsindex. Die Menge der Flüssigkeit in der Linsenkammer bestimmt die Brennweite der adaptiven Linse.

Bei einer alternativen Möglichkeit, dem Elektrowetting, wird die Linse mit zwei Flüssigkeiten unterschiedlicher optischer Eigenschaften gefüllt.

Bei einer weiteren vorteilhaften Ausführungsform der Erfindung ist das Autofokussystem von einer für HF-Felder undurchdringlichen HF-Abschirmeinrichtung umgeben. Dadurch werden die wenigen nicht-magnetischen, aber metallischen Komponenten, wie etwa Strom- und Datenkabel, vor dem HF-Feld abgeschirmt und eine unerwünschte Wechselwirkung vermieden. Wechselwirkungen können zum Beispiel durch das HF-Feld in den Komponenten erzeugte Ströme sein, die im ungünstigsten Fall die Komponente zerstören können.

Als Abschirmeinrichtung ist eine Metallfolie aus nicht-magnetischem Material, wie etwa Aluminium oder Kupfer, geeignet. Aber auch eine feste Umhüllung aus Aluminium oder eine metallische, nicht-magnetische Bedampfung der äußeren Oberflächen des Autofokussystems sind möglich.

Das Autofokussystem umfasst bei einer weiteren Ausführungsform der Erfindung eine Blende aus nicht-magnetischem Material sowie mindestens zwei achromatische Linsen.

Eine weitere Ausführungsform der Erfindung ist dadurch gekennzeichnet, dass das vom Messobjekt aus in das Kameraobjektiv eintretende Licht gegenüber der optischen Achse der Objektivlinsen einen Winkel von 90° einschließt, und dass das Licht anschließend über einen 45° - Spiegel aus nicht-magnetischem Material in die optische Ebene des Kameraobjektivs umgelenkt wird. Auf diese Weise ist eine besonders platzsparende Montage des Kameraobjektivs möglich. Die Baulänge des Kameraobjektivs ist bei dieser Ausführungsform nicht so relevant. Zudem führt eine Vergrößerung des Arbeitsabstands zur Verringerung der optischen Feldwinkel, was die Reduktion optischer Fehler zur Folge hat.

Eine alternative Ausführungsform sieht vor, dass das vom Messobjekt aus in das Kameraobjektiv eintretende Licht parallel zur optischen Achse der Objektivlinsen eintritt. Hier wird der Spiegel als optisches Element eingespart und so die Baulänge des Kameraobjektivs reduziert.

Es ist vorteilhaft, wenn eine elektronische Kontrolleinheit zur Steuerung oder Regelung des Autofokussystems vorgesehen ist. Die Kontrolleinheit wird vorteilhafterweise außerhalb der Bohrung vorgesehen sein und erfordert daher nicht notwendig die Verwendung von nicht-magnetischen Materialien. Mit geeigneter Abschirmung ist es jedoch auch denkbar, die Kontrolleinheit ebenfalls innerhalb der Bohrung des Magneten vorzusehen.

Weitere Vorteile der Erfindung ergeben sich aus der Beschreibung und der Zeichnung. Ebenso können die vorstehend genannten und die weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detailbeschreibung und Zeichnungen

Es zeigen:
- Fig. 1:: eine schematische räumliche Darstellung einer erfindungsgemäßen adaptiven Silikon-Membran-Linse;
- Fig. 2:: eine schematische Darstellung des optischen Aufbaus eines erfindungsgemäßen Kameraobjektivs inklusive Strahlengang des vom Messobjekt ausgehenden Lichts;
- Fig. 3:: eine räumliche schematische Darstellung einer Ausführungsform des erfindungsgemäßen Kameraobjektivs;
- Fig. 4:: eine schematische Darstellung der Anordnung einer Ausführungsform des erfindungsgemäßen Kameraobjektivs in einer MR-Apparatur, a) in linearer Bauweise ohne Spiegel und b) bei Verwendung eines 45° Spiegels wie in Fig.2.

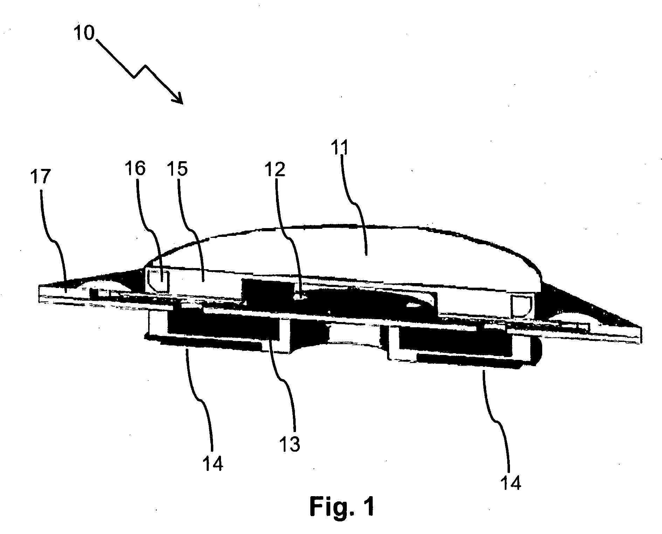

**Fig. 1** zeigt eine schematische räumliche Darstellung einer Ausführungsform der erfindungsgemäßen adaptiven **Silikon-Membran-Linse 10,** die ursprünglich für den Mobil-Elektronik Markt entwickelt wurde. Sie besteht aus zwei Komponenten: **Linsenkammer 12** und **Pumpkammer 13,** welche auf einem **Glassubstrat 17** miteinander verbunden sind.

Die Linsenkammer 12 ist mit einer optisch transparenten Flüssigkeit, insbesondere einem Immersionsöl, gefüllt und setzt sich aus einer **Silikonmembran 11** mit **Stützring 15** zusammen. Die Pumpkammer 13, in welcher sich auch die Linsenflüssigkeit befindet, besteht aus einem in Silikon eingebetteten **piezoelektrischen Biegewandler 14.**

Der Flüssigkeitsaustausch zwischen Pumpkammer 13 und Linsenkammer 12 wird über Öffnungen im Glassubstrat 17 erreicht. Wird am Piezo-Biegewandler 14 eine Spannung angelegt, so wölbt sich dessen innerer Bereich nach oben und verdrängt Linsenflüssigkeit aus der Pumpkammer 13 in die Linsenkammer 12. Das hat eine zunehmende Auswölbung der Silikon-Membran 11 und damit eine Verringerung der Linsenbrennweite zur Folge.

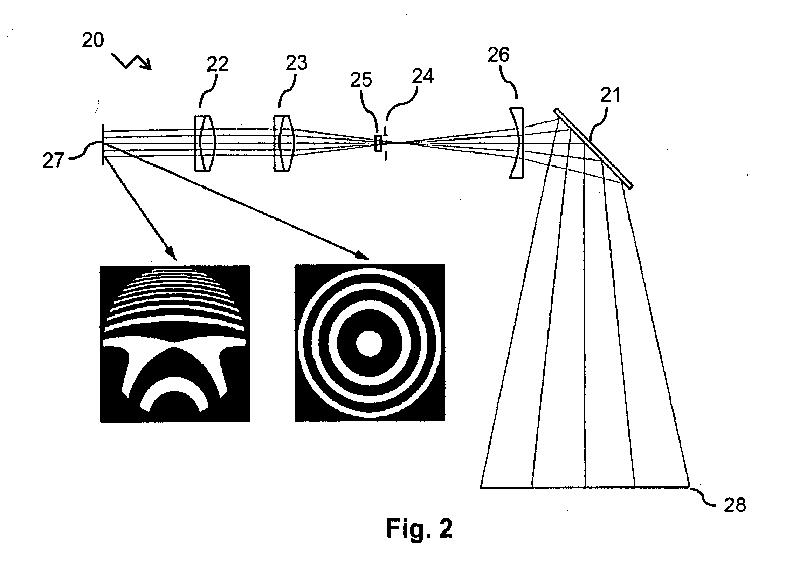

**Fig. 2** zeigt eine schematische Darstellung eines möglichen optischen Aufbaus des **Kameraobjektivs 20.** Das vom **Messobjekt 28** ausgehende Licht trifft auf einen im Winkel von 45° angeordneten **Spiegel 21** aus nicht-magnetischem Material. Dieser lenkt das einfallende Licht um etwa 90° in eine Richtung parallel zur optischen Achse des Linsensystems um. Das Licht passiert zunächst eine **plan-konkave Linse 26,** die das Licht fokussiert. Im Brennpunkt der plan-konkaven Linse 26 (oder kurz davor oder danach) ist eine **Blende 24** angeordnet. Eine **adaptive Linse 25** sorgt stets für eine optimale Abbildung des Messobjekts 28 auf die **Kamera 27,** die beispielsweise als CCD-Kamera ausgeführt sein kann. Zwei **achromatische Linsen 22, 23** sorgen dafür, dass das Licht parallel auf die Kamera 27 trifft.

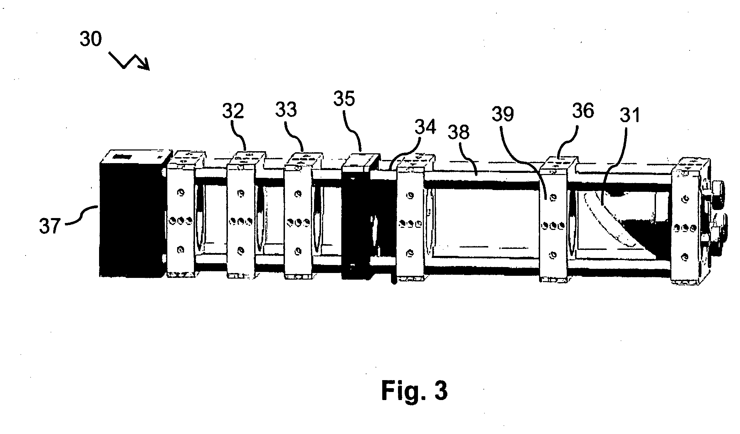

Eine mögliche Ausführung des **Kameraobjektivs 30** ist in **Fig. 3** schematisch räumlich dargestellt. Konkrete Randbedingungen für das Weitwinkelobjektiv in der gezeigten Ausführungsform sind eine Objektgröße von 60 x 4 mm², eine Bildgröße auf dem Kamerachip (6,54 x 5,23 mm²), die Objektweite (Abstand zwischen Objektiv und Objekt) und die Länge des Objektivs. Beim optischen Design des adaptiven Kameraobjektivs 30 wurde zur Unterstützung die Raytracing Software ZEMAX^{®} verwendet. Nach der theoretischen Optimierung des Objektivs wurde dieses aus Linos-Komponenten aufgebaut. Jede einzelne optische Komponente (also **Spiegel 31, plan-konkave Linse 36, Blende 34, adaptive Linse 35** und die **achromatischen Linsen 32, 33**) ist dabei an einer **Aufnahmeplatte 39** befestigt. Alle Aufnahmeplatten sind wiederum mit vier **Stangen 38,** z.B. aus Aluminium miteinander verbunden, an denen auch die **Kamera 37** befestigt ist.

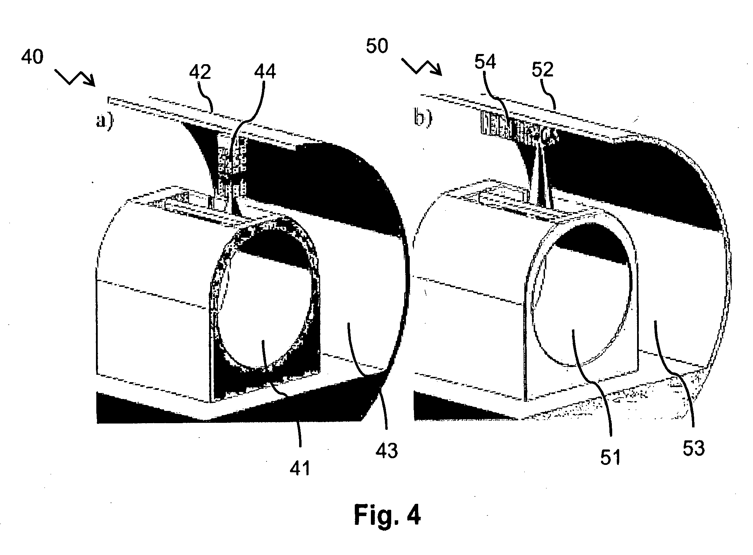

**Fig. 4** zeigt zwei mögliche Anordnungen des erfindungsgemäßen **Kameraobjektivs 44, 54** in der **Bohrung 43, 53** des **MR-Magneten 42, 52** einer **MR-Apparatur 40, 50.** Man beachte, dass das Kameraobjektiv 44, 54 zwar innerhalb der Bohrung 43, 53 des MR-Magneten 42, 52 angebracht ist, jedoch nicht in das **Messvolumen 41, 51,** in welchem das das Messobjekt positioniert wird, hineinragt.

Fig. 4 a) zeigt eine MR-Apparatur 40 mit einem linearen Kameraobjektiv 44, welches sich durch eine kleine Baulänge auszeichnet.

Die MR-Apparatur 50 in Fig. 4 b) umfasst ein Kameraobjektiv 54 mit Spiegel, wie es in Fig. 2 schematisch dargestellt ist. Dieses Kameraobjektiv zeichnet sich dadurch aus, dass es weniger in Richtung des Messvolumens 51 ausgedehnt ist und somit auch in MR-Magnetsystemen mit hohen Magnetfeldstärken und kleiner Bohrung angebracht werden kann, ohne in das Messvolumen hineinzuragen.

### Bezugszeichenliste:

| | | | |
|---|---|---|---|
| 10 | Adaptive Silikon-Membran-Linse | 30 | Kameraobjektiv |
| 11 | Silikon-Membran | 31 | Spiegel |
| 12 | Linsenkammer | 32 | Achromatische Linse |
| 13 | Pumpkammer | 33 | Achromatische Linse |
| 14 | Piezoelektrischer Biegewandler | 34 | Blende |
| 15 | Stützring | 35 | Adaptive Linse |
| 16 | Stabilisierungsring | 36 | Plan-konkave Linse |
| 17 | Glassubstrat | 37 | Kamera |
| | | 38 | Halte-Stangen |
| 20 | Kameraobjektiv | 39 | Aufnahmeplatte |
| 21 | Spiegel | | |
| 22 | Achromatische Linse (f=200 mm) | 40, 50 | MR-Apparatur |
| 23 | Achromatische Linse (f=40 mm) | 41, 51 | Messvolumen |
| 24 | Blende | 42, 52 | MR-Magnetsystem |
| 25 | Adaptive Linse | 43, 53 | Bohrung des MR-Magneten |
| 26 | Plan-konkave Linse (f=-40 mm) | 44 | Kameraobjektiv linear |
| 27 | Kamera | 54 | Kameraobjektiv mit Spiegel |
| 28 | Messobjekt | | |

## Patentansprüche

1. Magnetresonanz (=MR) Apparatur (40; 50) mit einem MR- Magnetsystem (42; 52) zur Erzeugung eines homogenen Magnetfelds, das eine Bohrung (43; 53) zur Aufnahme eines Messobjektes (28) aufweist, sowie mit einem optischen Bewegungsmonitorsystem zur Erfassung von Bewegungen des Messobjektes (28), umfassend eine innerhalb der Bohrung (43; 53) angeordnete, optische Kamera (27; 37) mit einem Kameraobjektiv (20; 30; 44; 54), sowie eine Vorrichtung zur Übertragung von Monitoringdaten an einen außerhalb des MR- Magnetsystems (42; 52) vorgesehenen Bildwandler,
**dadurch gekennzeichnet,**
**dass** das Kameraobjektiv (20; 30; 44; 54) ein Autofokussystem aus nicht-magnetischem Material umfasst,
**dass** das Autofokussystem Hochfrequenz (=HF) Felder weder absorbiert noch reflektiert noch emittiert,
**dass** im Autofokussystem mindestens eine adaptive Linse (25; 35) angeordnet ist,
**dass** die adaptive Linse (25; 35) eine Silikon-Membran-Linse (10) oder aus einem Voll-Polymer hergestellt ist, und
**dass** die adaptive Linse (25; 35) eine Linsenkammer (12), eine Pumpkammer (13) und einen piezoelektronischen Biegewandler (14) umfasst, die jeweils aus nicht-magnetischem Material hergestellt sind,
wobei zur Brennweiteneinstellung mittels des piezoelektrischen Biegewandlers (14) Flüssigkeit von der einen Kammer in die andere Kammer verdrängt werden kann.

2. Magnetresonanz-Apparatur nach Anspruch 1, **dadurch gekennzeichnet, dass** die adaptive Linse (25; 35) mit einer optisch transparenten Flüssigkeit gefüllt ist.

3. Magnetresonanz-Apparatur nach einem der vorhergehenden Ansprüche,
**dadurch gekennzeichnet, dass** das Autofokussystem von einer für HF-Felder undurchdringlichen HF-Abschirmeinrichtung umgeben ist.

4. Magnetresonanz-Apparatur nach einem der vorhergehenden Ansprüche,
**dadurch gekennzeichnet, dass** das Autofokussystem eine Blende (24; 34) aus nicht-magnetischem Material sowie mindestens zwei achromatische Linsen (22, 23; 32, 33) umfasst.

5. Magnetresonanz-Apparatur nach einem der Ansprüche 1 bis 4, **dadurch gekennzeichnet, dass** das Kameraobjektiv (20; 30; 54) mehrere Objektivlinsen enthält und dass das vom Messobjekt (28) aus in das Kameraobjektiv (20; 30; 54) eintretende Licht gegenüber der optischen Achse der Objektivlinsen (22, 23, 25, 26; 32, 33, 35, 36) einen Winkel von 90° einschließt, und dass das Licht anschließend über einen 45° - Spiegel (21; 31) aus nicht-magnetischem Material in die optische Ebene des Kameraobjektivs (20; 30; 54) umgelenkt wird.

6. Magnetresonanz-Apparatur nach einem der Ansprüche 1 bis 4, **dadurch gekennzeichnet, dass** das Kameraobjektiv (20; 30; 54) mehrere Objektivlinsen enthält und dass das vom Messobjekt (28) aus in das Kameraobjektiv (44) eintretende Licht parallel zur optischen Achse der Objektivlinsen (22, 23, 25, 26; 32, 33, 35, 36) eintritt.

7. Magnetresonanz-Apparatur nach einem der vorhergehenden Ansprüche, **dadurch gekennzeichnet, dass** eine elektronische Kontrolleinheit zur Steuerung oder Regelung des Autofokussystems vorgesehen ist.

## Claims

1. Magnetic resonance (MR) apparatus (40; 50) having
an MR magnet system (42; 52) for generating a homogeneous magnetic field, the MR magnet system comprising a bore (43; 53) for accommodating a test object (28),
and also having an optical motion monitoring system for detecting motions of the test object (28) comprising an optical camera (27; 37), which is arranged inside the bore (43; 53) and comprises a camera lens (20; 30; 44; 54), and also a device for transmitting monitoring data to an image converter provided outside of the MR magnet system (42; 52), **characterized in that**
the camera lens (20; 30; 44; 54) comprises an autofocus system of non-magnetic material,
the autofocus system neither absorbs nor reflects nor emits radio frequency (RF) fields,
at least one adaptive lens (25; 35) is arranged in the autofocus system, the adaptive lens (25; 35) is a silicone membrane lens (10) or is produced from full polymer, and
the adaptive lens (25; 35) comprises a lens chamber (12), a pump chamber (13) and a piezoelectric bending actuator (14), each being produced from a non-magnetic material, wherein liquid can be displaced from one chamber into the other chamber by means of the piezoelectric bending actuator for adjusting the focal length.

2. Magnetic resonance apparatus according to claim 1, **characterized in that** the adaptive lens (25; 35) is filled with an optically transparent liquid.

3. Magnetic resonance apparatus according to any one of the preceding claims, **characterized in that** the autofocus system is surrounded by an RF shielding device which is impermeable to RF fields.

4. Magnetic resonance apparatus according to any one of the preceding claims, **characterized in that** the autofocus system comprises an aperture (24; 34) of non-magnetic material as well as at least two achromatic lenses (22, 23; 32, 33).

5. Magnetic resonance apparatus according to any one of the claims 1 through 4, **characterized in that** the camera lens (20; 30; 54) contains a plurality of objective lenses and the light coming from the test object (28) and entering the camera lens (20; 30; 54) confines an angle of 90° with respect to the optical axis of the objective lenses (22, 23, 25, 26; 32, 33, 35, 36), and the light is subsequently deflected via a 45° mirror (21; 31) of non-magnetic material into the optical plane of the camera lens (20; 30; 54).

6. Magnetic resonance apparatus according to any one of the claims 1 through 4, **characterized in that** the camera lens (20; 30; 54) contains a plurality of objective lenses and the light coming from the test object (28) and entering the camera lens (44) is incident parallel with respect to the optical axis of the objective lenses (22, 23, 25, 26; 32, 33, 35, 36).

7. Magnetic resonance apparatus according to any one of the preceding claims, **characterized in that** an electronic control unit is provided for controlling or regulating the autofocus system.

## Revendications

1. Appareil (40 ; 50) à résonance magnétique (= RM) doté d'un système magnétique à résonance magnétique (42 ; 52) pour générer un champ magnétique homogène, qui présente un alésage (43 ; 53) pour recevoir un objet à mesurer (28), ainsi que d'un système optique de contrôle de déplacement pour détecter des mouvements de l'objet à mesurer (28), comprenant une caméra optique (27; 37) disposée à l'intérieur de l'alésage (43 ; 53), dotée d'un objectif de caméra (20 ; 30 ; 44 ; 54), ainsi qu'un dispositif pour la transmission de données de surveillance à un convertisseur d'image prévu à l'extérieur du système magnétique à résonance magnétique (42 ; 52),
**caractérisé en ce**
**que** l'objectif de caméra (20 ; 30 ; 44 ; 54) comprend un système d'autofocus en matériau non magnétique,
**que** le système d'autofocus n'absorbe, ne réfléchit ni n'émet de champs à haute fréquence (= HF),
**qu'**au moins une lentille adaptative (25 ; 35) est disposée dans le système d'autofocus,
**que** la lentille adaptative (25 ; 35) est une lentille à membrane silicone (10) ou est fabriquée à partir d'un polymère plein, et
**que** la lentille adaptative (25 ; 35) comprend une chambre de lentille (12), une chambre de pompage (13) et un transducteur piézoélectrique de flexion (14) qui sont tous fabriqués à partir d'un matériau non magnétique, sachant que pour régler la distance focale, du liquide peut être déplacé d'une chambre dans l'autre chambre au moyen du transducteur piézoélectrique de flexion (14).

2. Appareil à résonance magnétique selon la revendication 1, **caractérisé en ce que** la lentille adaptative (25 ; 35) est remplie d'un liquide transparent optiquement.

3. Appareil à résonance magnétique selon une des revendications précédentes, **caractérisé en ce que** le système d'autofocus est entouré par un dispositif de blindage HF impénétrable aux champs HF.

4. Appareil à résonance magnétique selon une des revendications précédentes, **caractérisé en ce que** le système d'autofocus comprend un diaphragme (24 ; 34) en matériau non magnétique ainsi qu'au moins deux lentilles achromatiques (22, 23 ; 32 ; 33).

5. Appareil à résonance magnétique selon une des revendications 1 à 4, **caractérisé en ce que** l'objectif de caméra (20 ; 30 ; 54) contient plusieurs lentilles d'objectif et que la lumière venant de l'objet à mesurer (28) qui entre dans l'objectif de caméra (20 ; 30 ; 54) fait un angle de 90° avec l'axe optique des lentilles d'objectif (22, 23, 25, 26 ; 32, 33, 35, 36), et que la lumière est ensuite déviée dans le plan optique de l'objectif de caméra (20 ; 30 ; 54) par un miroir à 45° (21 ; 31) en matériau non magnétique.

6. Appareil à résonance magnétique selon une des revendications 1 à 4, **caractérisé en ce que** l'objectif de caméra (20 ; 30 ; 54) contient plusieurs lentilles d'objectif et que la lumière venant de l'objet à mesurer (28) qui entre dans l'objectif de caméra (44) arrive parallèlement à l'axe optique des lentilles d'objectif (22, 23, 25, 26 ; 32, 33,35,36).

7. Appareil à résonance magnétique selon une des revendications précédentes, **caractérisé en ce qu'**une unité de contrôle électronique est prévue pour commander ou réguler le système d'autofocus.
